# FASCICULE DE BREVET EUROPEEN

(11) **EP 2 273 956 B1**
(45) Date de publication et mention de la délivrance du brevet: **28.01.2015**
(21) Numéro de dépôt: 09742221.6
(22) Date de dépôt: 20.03.2009
(51) Int. Cl.: A61F 5/01

(54) **ORTHÈSE DE CHEVILLE**
FUSSGELENKSCHIENE
ANKLE SPLINT

(30) Priorité: 05.05.2008 FR 0802474
(43) Date de publication de la demande: 19.01.2011
(73) Titulaire: Gibaud, 42000 Saint Etienne (FR)
(72) Inventeur: BECKERS, Thomas, 69630 Chaponost (FR); ANGLADA, Gérard, 42000 Saint Etienne (FR)
(74) Mandataire: Delorme, Nicolas
(86) Numéro de dépôt international: PCT/FR2009/050473
(87) Numéro de publication internationale: WO 2009/136023

(56) Documents cités:
- EP-A- 1 374 810
- CA-A1- 2 543 217
- FR-A- 961 981
- US-A- 2 477 591
- US-A- 4 572 170
- US-A1- 2004 260 220
- US-A1- 2007 142 760

## Description

La présente invention concerne une orthèse de cheville, plus particulièrement destinée au traitement de l'entorse du ligament latéral externe de la cheville.

On connaît déjà des orthèses utilisées pour ce traumatisme.

D'une part, le document EP 1 234 560 propose une orthèse de cheville comportant une coque en L présentant un montant en appui contre la jambe et une patte transversale placée sous le talon. Toutefois, la coque étant monolithique, cette orthèse sert essentiellement à immobiliser l'articulation de la cheville et n'est pas pleinement satisfaisante lorsqu'il s'agit de permettre à un utilisateur de marcher.

D'autre part, certaines orthèses sont conçues avec une articulation entre la partie placée contre la jambe et la partie placée contre le pied. Ainsi, ce type d'orthèse permet à un utilisateur de reprendre une activité progressivement, tout en continuant à assurer un certain maintien empêchant la réapparition du traumatisme. Cependant, cette orthèse munie d'un unique axe d'articulation présente notamment les inconvénients suivants :
- l'impact du pied au sol, lors de la marche, est transmis dans la jambe sans amortissement, ce qui est source d'inconfort et éventuellement d'aggravation du traumatisme ;
- pour obtenir un bon accompagnement des mouvements de la cheville, et donc un bon confort de l'utilisateur, il serait nécessaire de faire coïncider l'axe d'articulation de la cheville en flexion / extension avec l'axe d'articulation de l'orthèse. Or, en pratique, ceci est très difficile à réaliser, voire impossible.

Par ailleurs, le document US 2 477 591 propose une orthèse destinée à relever le pied de l'utilisateur, pour l'empêcher de butter sur le sol. Cette orthèse comporte une partie placée contre la jambe et une partie placée contre le pied, qui sont articulées l'une à l'autre par l'intermédiaire de deux axes de pivotement.

L'invention a pour but de remédier aux inconvénients mentionnés ci-dessus, en fournissant une orthèse de cheville visant à empêcher le varus, qui permette à un utilisateur de reprendre l'activité physique et lui offre un très bon confort d'utilisation.

D'autres orthèses de cheville sont décrites dans les documents EP 1 374 810 et FR 961 981. EP 1 374 810 décrit notamment une orthèse de cheville comportant une partie placée contre la jambe et une partie placée contre le pied, qui sont connectées de façon pivotante à une pièce de liaison intermédiaire élastique qui assure le rappel de l'orthèse dans sa position de repos.

A cet effet, l'invention concerne une orthèse de cheville conforme à la revendication 1.

On définit la position neutre d'utilisation de l'orthèse comme étant la position dans laquelle l'orthèse est fixée sur un utilisateur qui se tient debout (donc avec la jambe sensiblement verticale), en ayant la cheville en position neutre, c'est-à-dire ni en flexion ni en extension. Dans cette position, on définit la direction transversale comme la direction interne-externe de l'utilisateur.

En pratique, la pièce de liaison est située en regard de la malléole latérale et les deux axes d'articulation sont situés au voisinage de l'axe d'articulation de la cheville.

La présence sur l'orthèse de deux axes d'articulation décalés permet d'une part de générer un effet d'amortissement de l'impact du talon au sol lors de la marche, et d'autre part d'accompagner de façon beaucoup plus efficace les mouvements de flexion / extension de la cheville, en libérant l'articulation de la cheville. Ainsi, l'orthèse selon l'invention possède un confort de port nettement amélioré par rapport à l'art antérieur. De plus, elle permet à un utilisateur de reprendre une activité sportive tout en bénéficiant d'une tenue de sa cheville empêchant la réapparition du traumatisme.

De préférence, le deuxième axe d'articulation est décalé vers l'avant par rapport au premier axe d'articulation.

En position neutre d'utilisation, la coque et le montant du support peuvent s'étendre selon des directions sensiblement verticales décalées l'une par rapport à l'autre selon la direction antéropostérieure.

L'organe d'appui peut présenter une face interne en creux épousant sensiblement la forme de la malléole latérale. Cette forme, associée au fait que le matériau constitutif de l'organe d'appui est semi-rigide, contribue au confort de port de l'orthèse pour l'utilisateur.

L'orthèse peut en outre comporter une talonnette destinée à être assemblée à la patte du support et à être placée sous le pied.

Selon une réalisation possible, la talonnette comprend une semelle destinée à être placée sous le pied, qui présente une cavité transversale ouverte vers le bas, en position d'utilisation, ladite cavité étant agencée pour recevoir la patte du support de sorte que, en position montée, la face inférieure de la patte du support soit située sensiblement dans le même plan que la face inférieure de la semelle. Ainsi, le pied placé dans l'orthèse repose sur une face inférieure plane et sans surépaisseur.

Avantageusement, la semelle et la patte du support peuvent comprendre des moyens d'assemblage réciproques par emboîtement, tels que : au moins un plot d'une pièce s'engageant dans un trou ménagé dans l'autre pièce ; au moins un bossage s'étendant dans un plan horizontal (en position d'utilisation) sur une pièce et venant se loger dans une partie en creux correspondante de l'autre pièce ; etc.

La talonnette comprend par exemple un rebord latéral interne et/ou externe, et/ou un rebord postérieur, ce qui permet de limiter ou d'éviter totalement le déplacement du pied par rapport à la talonnette. Dans le cas où les trois rebords latéral interne, latéral externe et postérieur sont présents, la talonnette forme un logement pour le pied, et en particulier pour le talon, qui se trouve donc bien positionné et maintenu. En prévoyant une talonnette pourvue d'un logement de réception du talon, l'invention assure une très bonne stabilisation de la cheville, ce qui permet de ne munir l'orthèse que d'une coque (d'un seul côté de la jambe).

Néanmoins, l'orthèse peut comporter une coque supplémentaire destinée à être placée contre la face interne de la jambe, sensiblement en regard de la coque, en étant assemblée à cette coque par au moins une sangle.

On décrit à présent, à titre d'exemple non limitatif, un mode de réalisation possible de l'invention, en référence aux figures annexées :
La figure 1 est une vue en perspective d'une orthèse selon l'invention ;
La figure 2 est une vue latérale externe de cette orthèse, placée au niveau de la cheville d'un utilisateur ;
La figure 3 est une vue avant de cette orthèse, placée au niveau de la cheville d'un utilisateur ;
La figure 4 est une vue de dessous de cette orthèse ;
Les figures 5 à 9 sont des vues en perspective de différents éléments constitutifs de l'orthèse, à savoir respectivement : la coque, le support, la talonnette, l'organe d'appui externe, la pièce de liaison et la coque supplémentaire (interne).

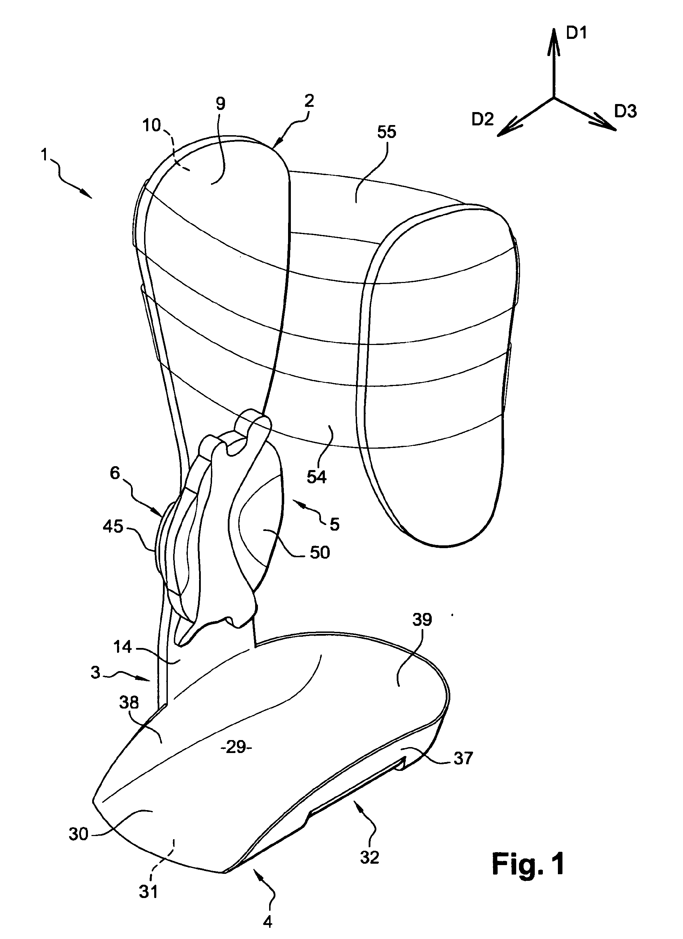

La figure 1 représente une orthèse 1 selon l'invention, qui comporte une coque 2 externe, un support 3, une talonnette 4, un organe d'appui externe 5, une pièce de liaison 6 et une coque 7 interne.

L'orthèse 1 va être décrite dans la position neutre d'utilisation, comme représenté sur les figures 1 à 4. On définit la direction verticale D1, la direction antéropostérieure D2 et la direction transversale D3 (interne-externe).

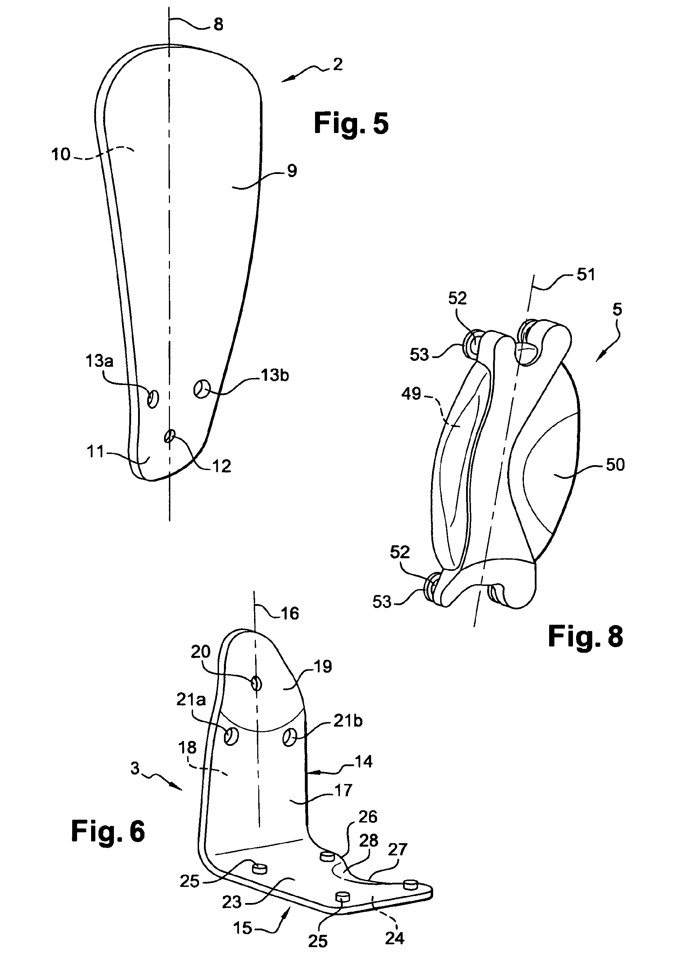

La coque 2, plus particulièrement illustrée sur la figure 5, est une pièce en matière rigide, par exemple en polyamide, qui est destinée à être placée contre la face externe de la jambe. La coque 2 possède une forme allongée verticalement et dont la largeur (selon D2) augmente de bas en haut. La coque 2 présente un axe de symétrie 8 vertical, une face interne 9 et une face externe 10. La face interne 9 présente une forme de type en « selle de cheval », c'est-à-dire possédant à la fois une forme incurvée autour d'une direction verticale, pour s'adapter à la forme globalement cylindrique de la jambe, et une forme bombée en partie inférieure, autour d'une direction antéropostérieure, créant une partie extrême inférieure 11 relevée vers l'extérieur, qui sera placée au niveau de la malléole latérale.

La coque 2 est en outre pourvue de trois orifices traversants : un orifice 12 situé sur l'axe de symétrie 8, en partie extrême inférieure 11, et deux orifices 13a, 13b situés au-dessus de l'orifice 12, de part et d'autre de l'axe de symétrie 8, et sensiblement alignés selon D2.

Le support 3, plus particulièrement illustré sur la figure 6, est une pièce en matière rigide, par exemple en polyamide, qui possède une forme en L comportant un montant 14 destiné à être placé contre la face externe du pied et une patte 15 destinée à être placée sous le pied. Le montant 14 et la patte 15 sont sensiblement perpendiculaires et raccordés par un congé.

Le montant 14 présente un axe de symétrie 16 vertical, une face interne 17 et une face externe 18. La partie extrême supérieure 19 du montant 14 est relevée vers l'extérieur et présente une forme incurvée autour d'une direction verticale, ceci afin de pouvoir être placée au niveau de la malléole latérale. Le montant 14 est en outre pourvu de trois orifices traversants : un orifice 20 situé sur l'axe de symétrie 16, en partie extrême supérieure 19, et deux orifices 21 a, 21 b situés au-dessous de l'orifice 20, de part et d'autre de l'axe de symétrie 16, l'orifice antérieur 21 a étant plus haut que l'orifice postérieur 21 b. On trouve également sur le montant 14 une nervure de renfort 22, faisant saillie verticalement du côté de la face externe 18.

La patte 15 est sensiblement plane et rectangulaire. Elle présente une face supérieure 23 et une face inférieure 24. De la face supérieure 23 font saillie, vers le haut, quatre plots 25 situés au voisinage des quatre coins de la patte 15. Le bord postérieur 26 de la patte 15 présente une zone en creux 27, disposée de façon sensiblement centrée entre deux plots 25. De plus, une dépression 28 globalement en forme de demi-disque est ménagée dans la patte 15, depuis la face supérieure 23, en regard de la zone en creux 27.

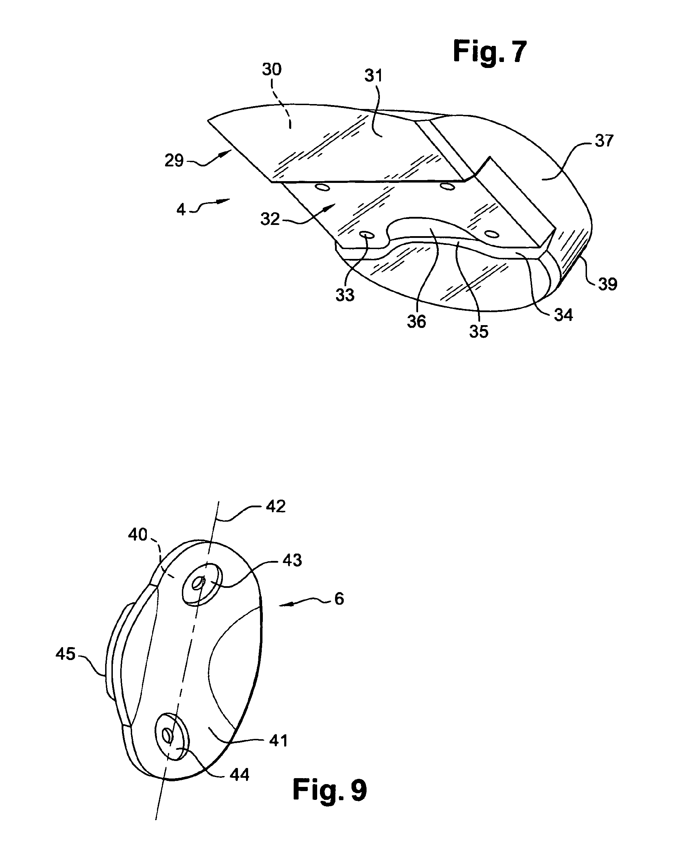

La talonnette 4, plus particulièrement illustrée sur la figure 7, est destinée à être assemblée au support 3 et à être placée sous le pied. Dans le mode de réalisation décrit ici, la talonnette 4 est réalisée en un matériau élastomère, ce qui permet d'obtenir un effet d'amorti et d'améliorer le confort de port de l'orthèse 1. Il est à noter que le matériau constitutif de la talonnette 4 peut également posséder des propriétés anti glisse. Ce matériau est, par exemple, un silicone ou du SEBS (styrène éthylbutylène styrène).

La talonnette 4 comprend une semelle 29 possédant une face supérieure 30 adaptée à la morphologie du pied et une face inférieure 31 sensiblement plane. Une cavité 32 s'étendant transversalement d'un côté à l'autre de la semelle 29 est ménagée dans la talonnette 4, depuis la face inférieure 31 de la semelle 29. Cette cavité 32, qui est donc ouverte vers le bas, est prévue pour recevoir la patte 15 du support 3, et possède donc une forme complémentaire de cette patte 15. Ainsi, la cavité 32 comporte quatre trous 33 dans lesquels les plots 25 peuvent venir s'emboîter, un bord postérieur 34 présentant une zone en relief 35 vers l'avant disposée de façon sensiblement centrée entre deux trous 33, et un bossage 36 sensiblement en forme de demi disque s'étendant vers l'avant et vers le bas depuis ladite zone en relief 35.

La patte 15 vient se positionner dans la cavité 32 et est maintenue en position par l'emboîtement des plots 25 dans les trous 33 et par la coopération entre la zone en relief 35 et la zone en creux 27, d'une part, et le bossage 36 et la dépression 28, d'autre part. Ainsi, le support 3 est parfaitement fixé à la talonnette 4. De plus, en position montée, la face inférieure 24 de la patte 15 et la face inférieure 31 de la semelle 29 sont situées sensiblement dans le même plan, si bien que le pied de l'utilisateur repose sur une surface plane sans surépaisseur.

La talonnette 4 comprend également un rebord latéral interne 37, un rebord latéral externe 38 et un rebord postérieur 39, qui forment un logement recevant le pied et le maintenant en position.

La pièce de liaison 6, plus particulièrement illustré sur la figure 9, est une pièce en matière rigide, par exemple en polyamide. Elle présente une forme de coupelle ayant une face externe 40 bombée et une face interne 41 en creux, et possède un axe de symétrie 42. La pièce de liaison 6 est pourvue de deux orifices traversants situés sur cet axe de symétrie 42, à savoir un orifice supérieur 43 et un orifice inférieur 44. Enfin, dans la réalisation représentée, la pièce de liaison 6 comporte des moyens d'attache 45 d'une sangle 46, disposés de part et d'autre de l'axe de symétrie 42, qui sont par exemple constitués par des organes faisant saillie vers l'extérieur et pourvus d'une fente de passage pour la sangle.

La pièce de liaison 6 est montée entre la coque 2 et le support 3, du côté interne, et assemblée à ces deux pièces au moyen d'organes tels que des rivets. Un premier rivet est ainsi engagé dans l'orifice 12 de la coque 2 et dans l'orifice supérieur 43 de la pièce de liaison 6, placés en correspondance l'un de l'autre. Un deuxième rivet est en outre engagé dans l'orifice 20 du support 3 et dans l'orifice inférieur 44 de la pièce de liaison 6, placés en correspondance l'un de l'autre.

De la sorte, la pièce de liaison 6 est montée pivotante d'une part par rapport à la coque 2 autour d'un premier axe d'articulation 47 transversal, correspondant à l'axe commun des orifices 12, 43, et d'autre part par rapport au support 3 autour d'un deuxième axe d'articulation 48 transversal, correspondant à l'axe commun des orifices 20, 44.

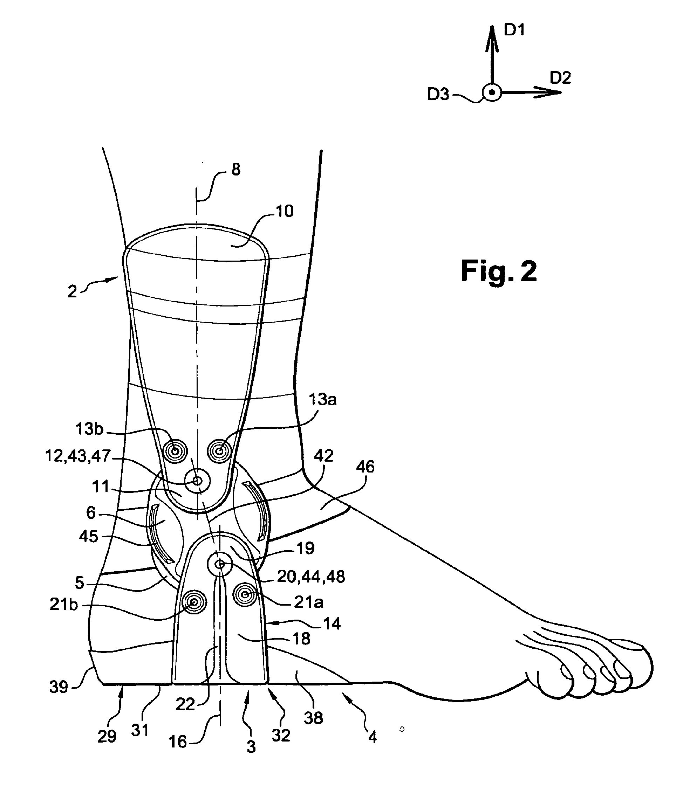

Comme on le voit sur la figure 2, en position neutre d'utilisation, l'axe de symétrie 8 de la coque 2 et l'axe de symétrie 16 du montant 14 du support 3 s'étendent selon des directions sensiblement verticales décalées l'une par rapport à l'autre selon la direction D2, l'axe de symétrie 8 de la coque 2 étant situé plus vers l'arrière. Ainsi, l'axe de symétrie 42 de la pièce de liaison 6 est incliné par rapport à la verticale, vers l'arrière et de bas en haut. L'angle d'inclinaison doit être adapté à la morphologie du pied. Il est par exemple de l'ordre de 10° à 20°. De plus, les premier et deuxième axes d'articulation 47, 48 sont décalés l'un par rapport à l'autre verticalement et selon la direction antéropostérieure (en position neutre d'utilisation), le deuxième axe d'articulation 48 étant situé plus vers l'avant et plus vers le bas.

L'organe d'appui externe 5, plus particulièrement illustré sur la figure 8, est fixé à la coque 2 et au support 3, en regard de la face interne 41 de la pièce de liaison 6, et est destiné à être placé contre la malléole latérale.

L'organe d'appui externe 5 présente une forme de coupelle ayant une face externe 49 bombée et une face interne 50 en creux, épousant sensiblement la forme de la malléole latérale. Il possède par ailleurs un axe de symétrie 51. L'organe d'appui externe 5 est pourvu de moyens d'assemblage à la coque 2 et au support 3, qui peuvent prendre toute forme adaptée. Dans la réalisation représentée, ces moyens d'assemblage sont constitués par des tétons 52 transversaux, dirigés vers le côté externe, munis à leur extrémité libre d'une collerette 53 de blocage. Ces tétons 52 sont répartis en un ensemble de deux tétons supérieurs, s'engageant dans les orifices 13a, 13b de la coque, et un ensemble de deux tétons inférieurs, s'engageant dans les orifices 21 a, 21 b du montant 14 du support 3.

L'organe d'appui externe 5 est réalisé en un matériau semi-rigide à propriétés élastomères, par exemple en silicone ou en SEBS.

La fonction de l'organe d'appui externe 5 est double. D'une part, cet organe d'appui 5 permet de maintenir la coque 2 et le support 3 dans la position neutre d'utilisation lorsque la cheville est en position neutre. D'autre part, cet organe d'appui 5 est agencé pour se déformer élastiquement lorsque la cheville est en flexion ou en extension, afin d'autoriser le pivotement de la pièce de liaison 6 autour des premier et deuxième axes d'articulation 47, 48, et pour rappeler l'orthèse 1 vers sa position neutre lorsque la cheville revient en position neutre.

Ainsi, on comprend que la rigidité de l'organe d'appui externe 5 doit être suffisante pour assurer les fonctions de maintien et de retour en position neutre. Mais l'organe d'appui externe 5 doit également être suffisamment souple pour pouvoir se déformer et ainsi autoriser le double pivotement de l'orthèse 1. De plus, la souplesse de l'organe d'appui externe 5 permet d'améliorer le confort de port de l'orthèse 1 par l'effet d'amorti procuré, et par le contact plus agréable contre la malléole latérale.

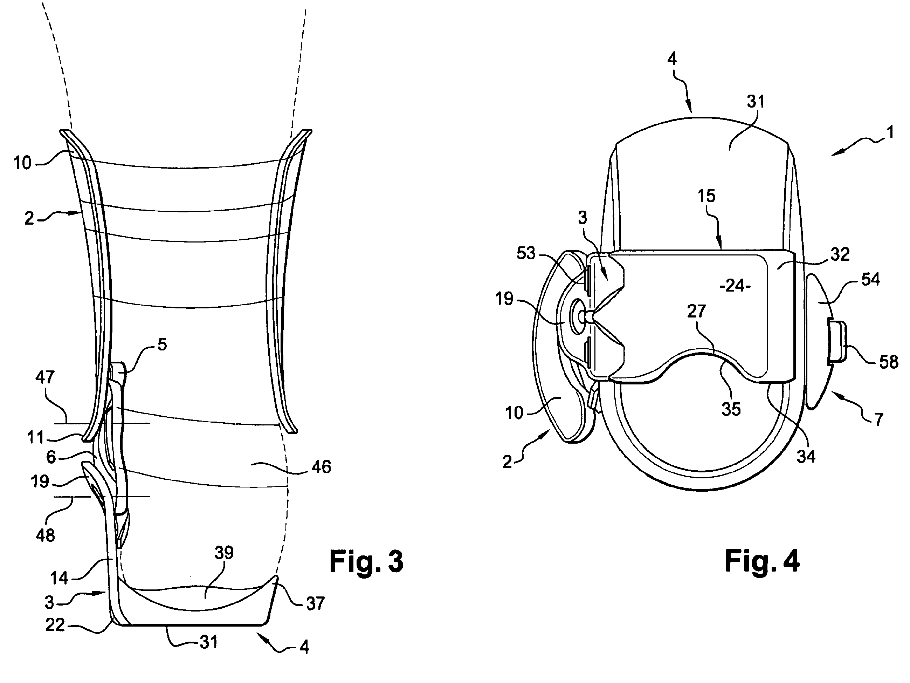

Enfin, on décrit la coque interne 7, plus particulièrement illustrée sur les figures 1 et 3. Il s'agit d'une pièce en matière rigide, par exemple en polyamide, qui est destinée à être placée contre la face interne de la jambe. La coque interne 7 possède sensiblement la même forme que la coque 2 interne, la présence d'orifices n'étant toutefois pas nécessaire.

Les deux coques 2, 7 sont assemblées l'une à l'autre par au moins une sangle, ici une sangle médiane 54 et une sangle supérieure 55. Des moyens d'attache de ces sangles peuvent être prévus sur l'une des coques ou chacune des coques 2, 7.

Selon une réalisation non représentée, il est possible de prévoir de plus un organe d'appui interne destiné à être placé contre la malléole médiale, et assemblé aux autres pièces constitutives de l'orthèse par une sangle (par exemple la sangle 46). Cet organe d'appui interne serait réalisé en matière semi rigide, par exemple en matériau élastomère tel qu'un silicone ou le SEBS et pourrait avantageusement présenter une forme de coupelle ayant une face externe bombée et une face interne en creux.

L'orthèse 1 est fixée sur la jambe et le pied de l'utilisateur par une sangle 46 pouvant entourer la zone située au voisinage de la cheville, en passant par les moyens d'attache 45 ménagés sur la pièce de liaison 6 et par les moyens d'attache 58 ménagés sur l'organe d'appui interne 7.

Cette orthèse 1 permet de libérer les mouvements de flexion / extension de la cheville, pour autoriser la marche et plus généralement la reprise d'une activité sportive. Le mouvement de valgus est également autorisé, dans la mesure où il n'a pas d'incidence négative sur le traumatisme du ligament latéral externe de la cheville. Ce mouvement est toutefois limité par la rigidité de l'orthèse 1.

En revanche, le mouvement de varus est fortement limité, car il pourrait engendrer une réapparition du traumatisme. Un faible varus est toutefois autorisé, pour que l'orthèse 1 puisse être portée par des utilisateurs ayant un léger varus naturel.

Il est à noter que l'orthèse 1 selon l'invention est peu encombrante, dans la mesure où elle ne comporte qu'une coque 2 (d'un seul côté de la jambe) et où la semelle 39 de la talonnette 4 ne s'étend que sous la moitié du pied environ. L'orthèse 1 peut donc être portée sans problème dans une chaussure.

Il va de soi que l'invention n'est pas limitée au mode de réalisation décrit ci-dessus à titre d'exemple mais qu'elle en embrasse au contraire toutes les variantes de réalisation. On pourrait notamment prévoir des butées limitant le déplacement relatif de la coque 2 et du support 3, et/ou une autre sangle de fixation de l'orthèse.

## Revendications

1. Orthèse de cheville comprenant :
- une coque (2) destinée à être placée contre la face externe de la jambe ;
- un support (3) en L comportant un montant (14) destiné à être placé contre la face externe du pied et une patte (15) destinée à être placée sous le pied ;
- une pièce de liaison (6) entre la coque (2) et le support (3), montée pivotante par rapport à la coque (2) autour d'un premier axe d'articulation transversal (47) et montée pivotante par rapport au support (3) autour d'un deuxième axe d'articulation transversal (48) ;
les premier et deuxième axes d'articulation (47, 48) étant décalés l'un par rapport à l'autre verticalement et selon la direction antéropostérieure, lorsque l'orthèse est placée sur la cheville d'un utilisateur, ladite cheville étant en position neutre,
l'orthèse (1) comprenant en outre un organe d'appui (5) disposé en regard de la face interne de la pièce de liaison (6) et fixé à la coque (2) et au support (3), ledit organe d'appui (5), réalisé en un matériau semi-rigide, étant agencé pour maintenir la coque (2) et le support (3) dans la position neutre d'utilisation lorsque la cheville est en position neutre, pour pouvoir être élastiquement déformé afin d'autoriser le pivotement de la pièce de liaison (6) autour des premier et deuxième axes d'articulation (47, 48), lorsque la cheville est en flexion ou en extension et, à partir de cette position déformée, pour rappeler l'orthèse (1) vers sa position neutre lorsque la cheville revient en position neutre.

2. Orthèse selon la revendication 1, dans laquelle le deuxième axe d'articulation (48) est décalé vers l'avant par rapport au premier axe d'articulation (47).

3. Orthèse selon la revendication 1 ou 2, dans laquelle en position neutre d'utilisation, la coque (2) et le montant (14) du support (3) s'étendent selon des directions (8, 16) sensiblement verticales décalées l'une par rapport à l'autre selon la direction antéropostérieure.

4. Orthèse selon l'une des revendications 1 à 3, dans laquelle l'organe d'appui (5) présente une face interne (50) en creux épousant sensiblement la forme de la malléole latérale.

5. Orthèse selon l'une des revendications 1 à 4, comprenant en outre une talonnette (4) destinée à être assemblée à la patte (15) du support (3) et à être placée sous le pied.

6. Orthèse selon la revendication 5, dans laquelle la talonnette (4) est réalisée en un matériau élastomère.

7. Orthèse selon la revendication 5 ou 6, dans laquelle la talonnette (4) comprend une semelle (29) destinée à être placée sous le pied, qui présente une cavité (32) transversale ouverte vers le bas, en position d'utilisation, ladite cavité (32) étant agencée pour recevoir la patte (15) du support (3) de sorte que, en position montée, la face inférieure (24) de la patte (15) du support (3) soit située sensiblement dans le même plan que la face inférieure (31) de la semelle (29).

8. Orthèse selon l'une des revendications 5 à 7, dans laquelle la semelle (29) et la patte (15) du support (3) comprennent des moyens d'assemblage réciproques par emboîtement (25, 27, 28, 33, 35, 36).

9. Orthèse selon l'une des revendications 5 à 8, dans laquelle la talonnette (4) comprend un rebord latéral interne (37) et/ou externe (38), et/ou un rebord postérieur (39).

10. Orthèse selon l'une des revendications 1 à 9, dans laquelle la pièce de liaison (6) comporte des moyens d'attache (45) d'une sangle.

11. Orthèse selon l'une des revendications 1 à 10, comprenant en outre une coque (7) supplémentaire destinée à être placée contre la face interne de la jambe, sensiblement en regard de la coque (2) en étant assemblée à cette coque (2) par au moins une sangle (54, 55).

## Patentansprüche

1. Knöchelorthese, die Folgendes umfasst:
- eine Schale (2), die dazu vorgesehen ist, gegen die Außenseite des Unterschenkels platziert zu werden;
- eine L-förmige Stütze (3), die einen Ständer (14), der dazu vorgesehen ist, gegen die Außenseite des Fußes platziert zu werden, und eine Lasche (15) umfasst, die dazu vorgesehen ist, unter dem Fuß platziert zu werden;
- ein Verbindungsteil (6) zwischen der Schale (2) und der Stütze (3), das in Bezug auf die Schale (2) drehbar um eine erste Gelenkquerachse (47) montiert ist und in Bezug auf die Stütze (3) drehbar um eine zweite Gelenkquerachse (48) montiert ist;
wobei die erste und die zweite Gelenkachse (47, 48) zueinander vertikal und gemäß der anteroposterioren Richtung versetzt sind, wenn die Orthese auf dem Knöchel eines Benutzers platziert ist, wobei der besagte Knöchel sich in der neutralen Position befindet,
wobei die Orthese (1) außerdem ein Auflageelement (5) umfasst, das gegenüber der Innenseite des Verbindungsteils (6) angeordnet und an der Schale (2) und der Stütze (3) befestigt ist, wobei das besagte Auflageelement (5), das aus einem halbsteifen Material hergestellt ist, dazu eingerichtet ist, die Schale (2) und die Stütze (3) in der neutralen Gebrauchsposition zu halten, wenn der Knöchel sich in der neutralen Position befindet, um sich elastisch verformen zu können, um das Schwenken des Verbindungsteils (6) um die erste und die zweite Gelenkachse (47, 48) zuzulassen, wenn der Knöchel sich in der Flexion oder der Extension befindet, und um aus dieser verformten Position die Orthese (1) in ihre neutrale Position zurückzusetzen, wenn der Knöchel in die neutrale Position zurückkehrt.

2. Orthese nach Anspruch 1, wobei die zweite Gelenkachse (48) in Bezug auf die erste Gelenkachse (47) nach vorne versetzt ist.

3. Orthese nach Anspruch 1 oder 2, wobei in der neutralen Gebrauchsposition die Schale (2) und der Ständer (14) der Stütze (3) sich in im Wesentlichen vertikalen Richtungen (8, 16) erstrecken, die zueinander gemäß der anteroposterioren Richtung versetzt sind.

4. Orthese nach einem der Ansprüche 1 bis 3, wobei das Auflageelement (5) eine vertiefte Innenseite (50) aufweist, die sich im Wesentlichen der Form des lateralen Knöchels anpasst.

5. Orthese nach einem der Ansprüche 1 bis 4, die außerdem eine Ferseneinlage (4) umfasst, die dazu vorgesehen ist, mit der Lasche (15) der Stütze (3) zusammengebaut und unter dem Fuß platziert zu werden.

6. Orthese nach Anspruch 5, wobei die Ferseneinlage (4) aus einem elastomeren Material hergestellt ist.

7. Orthese nach Anspruch 5 oder 6, wobei die Ferseneinlage (4) eine Sohle (29) umfasst, die dazu vorgesehen ist, unter dem Fuß platziert zu werden und die eine quer verlaufende Aushöhlung (32) aufweist, die in der Gebrauchsposition nach unten offen ist, wobei die besagte Aushöhlung (32) dazu eingerichtet ist, die Lasche (15) der Stütze (3) derart aufzunehmen, dass in der montierten Position die Innenseite (24) der Lasche (15) der Stütze (3) sich im Wesentlichen auf derselben Ebene wie die Innenseite (31) der Sohle (29) befindet.

8. Orthese nach einem der Ansprüche 5 bis 7, wobei die Sohle (29) und die Lasche (15) der Stütze (3) Mittel zum wechselseitigen Zusammenbau durch Ineinanderstecken (25, 27, 28, 33, 35, 36).

9. Orthese nach einem der Ansprüche 5 bis 8, wobei die Ferseneinlage (4) einen inneren seitlichen Rand (37) und/oder einen äußeren seitlichen Rand (38) und/oder einen hinteren Rand (39) umfasst.

10. Orthese nach einem der Ansprüche 1 bis 9, wobei das Verbindungsteil (6) Mittel zum Anbringen (45) eines Gurts umfasst.

11. Orthese nach einem der Ansprüche 1 bis 10, die außerdem eine zusätzliche Schale (7) umfasst, die dazu vorgesehen ist, gegen die Innenseite des Unterschenkels im Wesentlichen gegenüber der Schale (2) platziert zu werden, indem sie mit dieser Schale (2) mittels mindestens eines Gurts (54, 55) zusammengebaut wird.

## Claims

1. Ankle splint comprising:
- a shell (2) intended to be positioned against the external face of the leg;
- an L-shaped support (3) comprising an upright (14) intended to be positioned against the external face of the foot and a tongue (15) intended to be positioned under the foot;
- a connecting piece (6) connecting the shell (2) and the support (3), said connecting piece being mounted such that it can pivot with respect to the shell (2) about a first transverse axis of articulation (47) and mounted such that it can pivot with respect to the support (3) about a second transverse axis of articulation (48);
the first and second axes of articulation (47, 48) being offset from one another vertically and in the anteroposterior direction when the splint is positioned on the ankle of a user, said ankle being in the neutral position;
the splint (1) further comprising a bearing member (5) arranged opposite the internal face of the connecting piece (6) and attached to the shell (2) and to the support (3), said bearing member (5), made from a semi-rigid material, being designed to keep the shell (2) and the support (3) in the neutral position of use when the ankle is in the neutral position, to be able to be elastically deformed so as to allow the pivoting of the connecting piece (6) about the first and second axes of articulation (47, 48) when the ankle is in flexion or in extension and, from this deformed position, to return the splint (1) to its neutral position when the ankle returns to the neutral position.

2. Splint according to claim 1, wherein the second axis of articulation (48) is offset towards the front relative to the first axis of articulation (47).

3. Splint according to claim 1 or 2, wherein, in the neutral position of use, the shell (2) and the upright (14) of the support (3) extend in substantially vertical directions (8, 16) which are offset from one another in the anteroposterior direction.

4. Splint according to one of claims 1 to 3, wherein the bearing member (5) has a concave internal face (50) which substantially mates with the shape of the lateral malleolus.

5. Splint according to one of claims 1 to 4, further comprising a heel cushion (4) intended to be assembled to the tongue (15) of the support (3) and to be positioned under the foot.

6. Splint according to claim 5, wherein the heel cushion (4) is made from an elastomeric material.

7. Splint according to claim 5 or 6, wherein the heel cushion (4) comprises a sole (29) intended to be positioned under the foot, which sole has a transverse cavity (32) that is open in the downward direction in the position of use, said cavity (32) being designed to receive the tongue (15) of the support (3) so that, in the mounted position, the lower face (24) of the tongue (15) of the support (3) is located substantially in the same plane as the lower face (31) of the sole (29).

8. Splint according to one of claims 5 to 7, wherein the sole (29) and the tongue (15) of the support (3) comprise means for reciprocal assembly by engagement (25, 27, 28, 33, 35, 36).

9. Splint according to one of claims 5 to 8, wherein the heel cushion (4) comprises an internal lateral flange (37) and/or external lateral flange (38) and/or a posterior flange (39).

10. Splint according to one of claims 1 to 9, wherein the connecting piece (6) comprises means (45) for attaching a strap.

11. Splint according to one of claims 1 to 10, further comprising an additional shell (7) intended to be positioned against the internal face of the leg, substantially opposite the shell (2), by being assembled to this shell (2) by at least one strap (54, 55).
